# EUROPEAN PATENT SPECIFICATION

(11) **EP 3 254 731 B1**
(45) Date of publication and mention of the grant of the patent: **03.07.2019**
(21) Application number: 16173437.1
(22) Date of filing: 08.06.2016
(51) Int. Cl.: A61N 7/02, A61B 34/00, A61N 7/00, A61B 90/00

(54) **MULTI-PURPOSE ROBOTIC SYSTEM FOR MRI GUIDED FOCUSED ULTRASOUND TREATMENT**
MEHRZWECKROBOTERSYSTEM ZUR MRT-GEFÜHRTEN FOKUSSIERTEN ULTRASCHALLBEHANDLUNG
SYSTÈME ROBOTIQUE MULTIFONCTION POUR TRAITEMENT PAR ULTRASONS FOCALISÉS GUIDÉ PAR IRM

(43) Date of publication of application: 13.12.2017
(73) Proprietor: Cyprus University of Technology, 3603 Limassol (CY)
(72) Inventor: Damianou, Christakis, 4103 Limassol (CY); Yiannakou, Marinos, 3025 Limassol (CY)
(74) Representative: Soteriou-Loizidou, Lina

(56) References cited:
- WO-A2-2010/057067
- US-A1- 2011 028 867

## Description

### TECHNICAL FIELD

The present invention relates to a magnetic resonance imaging (MRI) compatible positioning system that carries a focused ultrasound transducer (FUS). The robotic system can be used for treating fibroid, abdominal, breast and brain cancer.

### BACKGROUND ART

Focused ultrasound (FUS) at high intensity and sufficient duration induces thermal changes in tissue. FUS is utilized to selectively heat biological tissues for oncological applications. FUS was explored in many targets accessible by ultrasound (eye 1, prostate 2, liver 3, brain 4-5, kidney 6-8 and breast 9).

FUS is either guided by ultrasound or MRI. Ultrasonic imaging is the most affordable method to guide FUS, however MRI offers superior contrast among tissues than ultrasound. Additionally MRI can effectively monitor the temperature changes produced by FUS.The main disadvantage of MRI is that is more expensive.

In order to treat large tissue volumes the FUS transducer should be navigated to create overlapping lesions. The robotic system responsible for such motion must be placed in the bore of the MRI scanner. The combination of ultrasound technology and MRI was first reported by Jolesz and Jakab 1991 10. The idea of using MRI to guide FUS technology was introduced in 1993 by Hynyen 11. In this study it was showed that lesions produced by FUS on a canine muscle *in vivo* were clearly visible with MRI.

The MRI guided FUS robotic systems developed in the subsequent years 12, 13 used hydraulic principles. Unfortunately, the hydraulic positioning systems had serious repeatability and reliability problems. Therefore, the actuation using hydraulic pricniples was abandoned. The motors used in the hydraulic robots interfered with the MRI, and therefore were placed far from the MRI scanner.

The robotic system described in patent 14 uses piezoelectric motors to move the transducer. This technology which was produced by InSightec was the first commercial MRI guided FUS system for the treatment of uterine fibroids. This system received the Food and Drug Administration (FDA) approval in 2004. The system has been used predominantly for the treatment of uterine fibroids 14. In their system the patient is placed in the prone position in the MRI. Since the patient is placed in prone position, and the treatment last for 3-4 hours, this is very uncomfortable for the patients.

Sonalleve MR-FUS is also a commercial system by Philips Healthcare Philips Healthcare, Netherland [16] which is used for fibroid treatment and bone palliation. In their system the patient is also placed in the prone position in the MRI.

Chopra et al 2009 17 developed an MRI-compatible, computer-controlled three-axis positioning system that delivers focused ultrasound in small animals. This system is used for preclinical studies. Each axis was constructed from custom-made nonmagnetic linear ball stages driven by piezoelectric actuators and optical encoders.

The MR guided FUS robotic system proposed by Damianou et al. [18] has three DoF and is small and portable and it can be seated on the table of any MRI scanner. The intended application of this positioning device is to conduct animal experiments.

Since the 1990s, FUS has been investigated to treat benign prostate hyperplasia (BPH) and prostate cancer. Several groups have conducted early feasibility studies on FUS ablation on prostate using a transrectal probe with dual capability of imaging and therapy 19, 20.

In 1992, preliminary results of in vivo effects of FUS on prostate carcinoma in rats were published, which suggested the use of FUS for the treatment of small localized prostate malignant tumors 20. Later on, Madersbacher and colleagues 21 investigated the potential of FUS on prostate cancer. Several clinical trials were reported thereafter 22, 23. Two transrectal devices, HIFU Sonablate 500 (Focal Surgery, Milpitas, CA now SonaCare Medical, Charlotte, North Carolina) and Ablatherm (Technomed International, Lyon, France), are the two key technologies for US guided FUS for prostate cancer.

This proposed robotic device incorporates only MRI compatible materials such as piezoelectric ultrasonic motors (Shinsei USR60E3N), Acrylonitrile Butadiene Styrene (ABS) plastic, and brass screws. Four PC controlled axes are needed: 3 linear and one angular (Θ). The system is manufactured automatically using a rapid prototyping system (FDM400, Stratasys, 7665 Commerce Way, Eden Prairie, Minnesota, 55344, USA). The patient can be positioned in supine position for all applications (breast, abdominal, brain, fibroids).

Furthermore, patent documents WO 2010/057067 and US 2011/028867 describe FUS devices for therapeutic use.

The invention is defined in the claims, other embodiments being merely exemplary.

### TECHNICAL PROBLEM TO BE SOLVED

In the area of the MRI guided FUS, for each organ, there is a specialized device. With the proposed system, multiple organs can be treated. In the future, more organs can be trated with the proposed system.

### DISCLOSURE OF THE INVENTION

The current invention relates to an MRI compatible postioning system which is capable of carrying a FUS transdcuer in order to treat diseases of the fibroid, abdominal, breast and brain.

The positioning system employs only MRI compatible materials such as piezoelectric motors and ABS plastic. The robotic system moves the transducer in 4 axes. The rotational motion of the piezoelectric motors is coupled to a jack screw gear mechanism. The piezo-electric motors that operate inside the MRI scanner minimally affect the quality of the MRI scanner because are placed at a distance from the MRI coil.

The positioning device is placed on the table of the MRI scanner and access of ultrasound to the abdominal, breast and brain is achieved from bottom to top. Sideway access to the brain is also possible. For fibroid the coupling can be a top to bottom approach. Top to bottom coupling can be applied also for breast. This is the first system that provides access to targets top to bottom, bottom to top and sideways. Because the height of the robotic system is small, this device can be incorporated inside the MRI table. With the use of a cushion the patients can be comfortably placed on this device. The movement of the device is very accurate due to the MR compatible encoders used.

Since the positioning device is placed on the table of the MRI scanner, this device can be used in all the available MRI scanners (ie it is a universal positioning device). The existing systems are placed inside the table of the MRI scanner, and therefore the system geometry has to altered depending on the type of MRI scanner. The device maintains high standards of repeatability and readability.

Another advantage of this positioning device is that it is lightweight and therefore it can be transported from one MRI scanner to another (ie it is considered portable).

### BRIEF DESCRIPTION OF THE DRAWINGS

Fig.1 Base of the robotic system that carries the X-stage.
Fig.2 X- stage of the robotic system that carries the Y-stage.

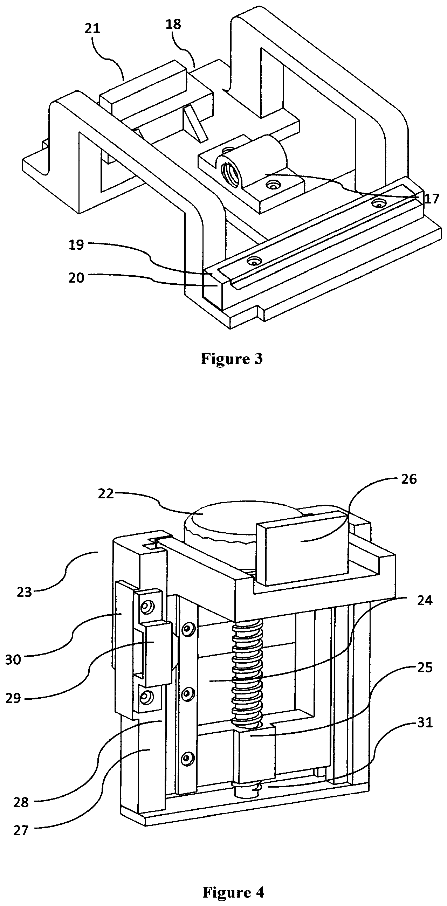

Fig.3 Y-stage of the robotic system.
Fig.4 Z-stage of the robotic system.
Fig.5 Theta-stage of the robotic system(plane perpendicular to the base).
Fig. 6 Theta-stage of the robotic system (plane parallel to the base).
Fig.7 Complete robotic system with all stages with no cover for the various motion mechanisms.
Fig.8 Complete robotic system with all stages and with cover of the various motion mechanisms (front view).
Fig.9 Complete robotic system with all stages and with cover of the various motion mechanisms (back view).
Fig. 10 Robotic system as attached to the brain (lateral coupling).
Fig.11 Robotic system as attached to the brain (bottom to top coupling).
Fig. 12 Robotic system as attached to the breast brain (bottom to top and lateral coupling).
Fig. 13 Robotic system as attached to the fibroids (top to bottom and lateral coupling).

### DESCRIPTION OF AT LEAST ONE WAY OF CARRYING OUT THE INVENTION

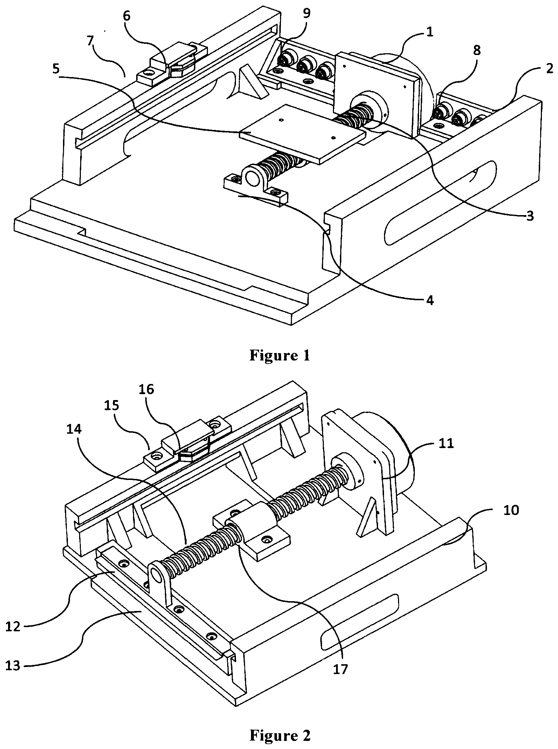

The X motor 1 is fixed on the motor holder of the base 2. Then the jack screw 3 is fitted to the X-motor shaft. The bracket 4 is designed to fit in the slot at the middle of the base 2 as shown in figure 1. The bracket 4 was added to ensure that the jack screw is centered during its rotation for smooth positioning of the X-plate. The bracket 4 is fixed in the slot of the base 2 with two brass screws. The bracket 5 attaches under the X-plate and it is forcing it to follow. On the top of the base 2 the encoder 6 and encoder holder 7 are fixed to provide accurate positioning of the X-plate. The motor connector holder 8 secures the motor connectors on the base 2. The encoder connector holder 9 secures the encoder connectors to the other side of the base 2.

The X-plate 10 includes a motor 11 which is attached to the motor holder for moving the Y-stage. The encoder strip holder 12 of the X-stage was designed to secure the encoder strip 13 and the support end of the Y-stage jack screw 14. Then the jack screw 14 is coupled to the motor shaft. The encoder holder 15 secures the optical encoder module 16 to the X-plate 10 to ensure the motion accuracy of the Y-stage. The bracket 17 attaches to the Y-plate 18 to convert the rotational to linear for the motion of the Y-stage.

The encoder strip holder 19 is attached to the Y-plate 18 to secure the linear encoder strip 20. The bracket 17 is fixed on the Y-stage. Two bridges were added on each side of the Y-plate to reinforce the middle section of the Y-plate 18. The middle section of the Y-plate 18 is narrower to provide enough room for the motion. The Y-plate 18 includes a coupling 21 to attach the Z-stage.

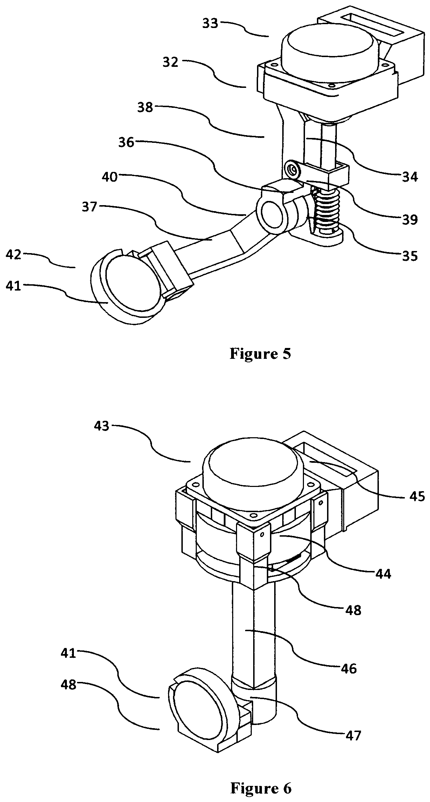

The Z-stage includes a motor 22 mounted on the motor holder of the frame 23. This motor 22 is coupled with the jack screw 24 below. The jack screw 24 is coupled to the lower section of the Z-plate 25. The Z-plate 25 includes a coupling 26 on the upper section to attach the theta stage. On the right side the encoder strip 27 and encoder strip holder 28 are mounted. An optical encoder 29 and an encoder holder 30 are used to accurately move the Z-stage. At the bottom of the frame 23 the bracket 31 is attached to ensure the jack screw rotates centered and to reinforce the frame 23. The theta stage shown in figure 5 is designed to maneuver the FUS transducer in a specific plane. On the motor holder 32 attaches a motor 33 that is coupled with the shaft 34. The shaft 34 includes a thread 35 on the one end. The thread 35 of the shaft 34 is coupled to a gear 36 that moves the lower section of the arm 37. The upper arm section 38 is integrated to the motor holder 32. A bracket 39 is fixed to the upper arm section to ensure a good coupling between the thread 35 and the gear 36. An axle 40 was designed to join the lower arm section 37 with the upper arm section 38. A transducer 41 is attached to the transducer holder 42 which is coupled to the lower arm section 37. An optical encoder inside the motor holder 32 is used for accurate motion of the theta stage.

Another theta mechanism was developed to enable the ultrasound to access targets through the front of the robot as shown in figure 6. This theta mechanism has the ability to rotate the transducer 360° around its vertical axis. The motor 43 is attached to the speed reduction unit 44. The reduction unit has a 64:1 reduction ratio which slows the motion for smooth motion. The speed reduction unit 44 includes a coupling 45 that attaches to the Z-stage. The motion of the reduction unit is transferred through an axle inside the upper arm 46 to the lower arm 47. The lower arm 47 includes a coupling that attaches to the transducer holder 48. Between the reduction unit 44 and the axle an optical encoder 48 is used for accurate motion.

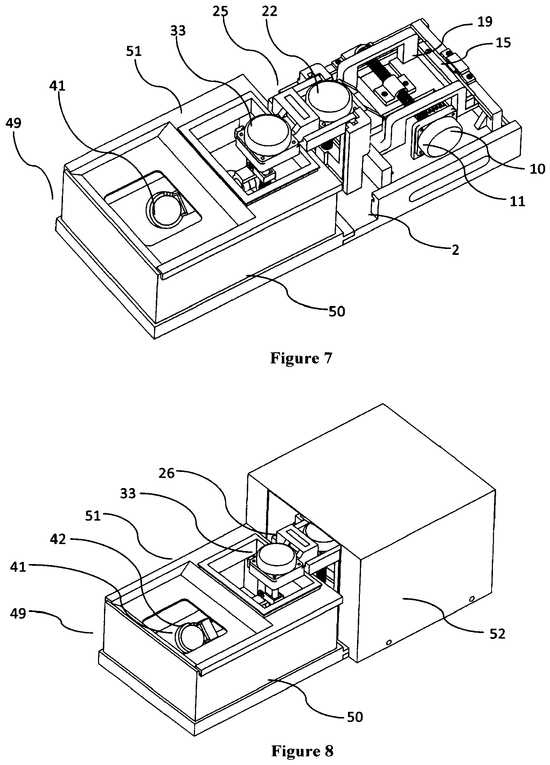

The positioning device shown in figures 7-9 navigates the transducer 41 inside the water container 49. The water container 49 is secured to the positioning device by the water container holder 50. The water container holder 50 is coupled to the base 2. A water container cover 51 which includes two openings is placed on the water container 50. It has two openings; the front allows FUS to access the target. A cover 52 hides the mechanisms of the positioning device.

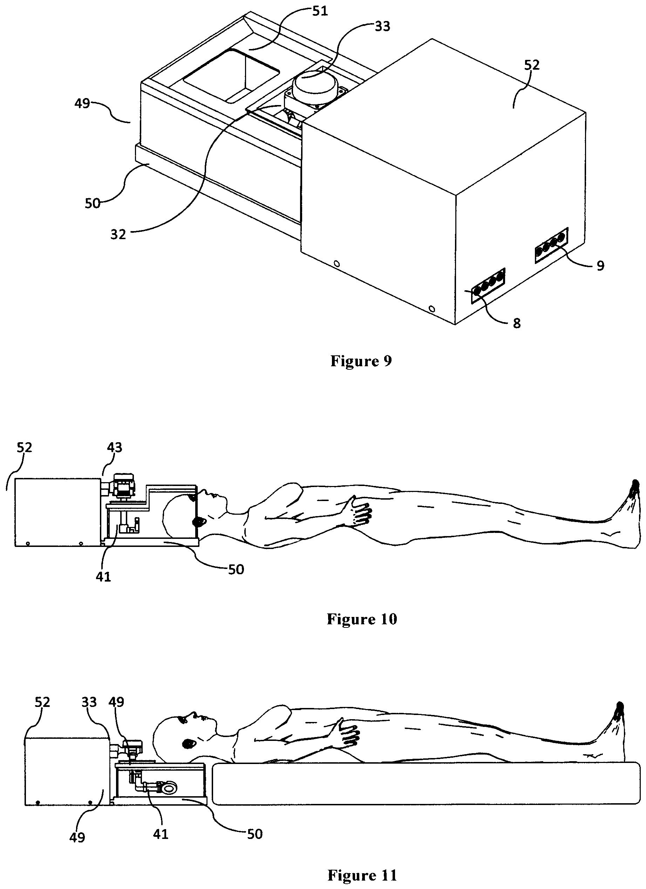

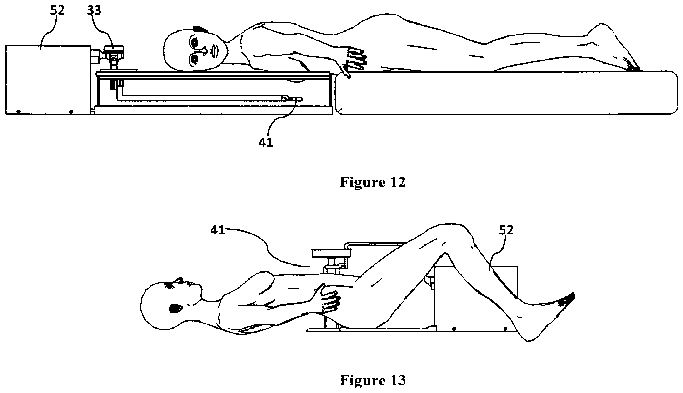

Fig.7 shows the complete robotic system with all stages with no cover showing the various motion mechanisms. Fig.8 shows the complete robotic system including a cover that hides the various motion mechanisms (front view). Fig.9 shows the complete robotic system with cover (back view). Fig. 10 shows the robotic system as attached to the brain (lateral coupling). Fig.11 shows the robotic system as attached to the brain (bottom to top coupling). Fig. 12 shows the robotic system as attached to the breast brain (bottom to top and lateral coupling). This arrangement can be used also for the abdominal area. Fig. 13 shows the robotic system as attached to the fibroids (top to bottom and lateral coupling). This arrangement can be used also for the abdominal area and breast.

### REFERENCES

1. Lizzi F, Coleman J, Driller J, Franzen L, Jakobiec F. Experimental, ultrasonically induced lesions in the retina, choroid, and sclera. Invest. Ophthalmol. Visual Sci. 1978, 205:350-360.
2. Chapelon JY, Margonari J, Vernier F, Gorry F, Ecochard R, Gelet A. In vivo effects of high-intensity ultrasound on prostatic adenocarcinoma Dunning R3327. Cancer Res. 1992; 52(22):6353-7.
3. ter Haar G, Sinnett D, Rivens I. High intensity focused ultrasound - a surgical technique for the treatment of discrete liver tumors. Phy. Med. Biol. 1989; 34(11):1743-50.
4. Lele PP. A simple method for production of trackless focal lesions with focused ultrasound. J. Physiol. 1962; 160:494-512.
5. Vykhodtseva NI, Hynynen K, Damianou C. Pulse duration and peak intensity during focused ultrasound surgery: theoretical and experimental effects in rabbit brain in vivo. Ultrasound Med Biol. 1994; 20(9):987-1000.
6. Linke C, Carteensen EL, Frizzell LA. Elbdawi A, Fridd CW. Localized Tissue destruction by High Intensity Focused Ultrasound. Arch. Surg. 1973; 107(6):887-891.
7. Hynynen K, Damianou CA Colucci V, Unger E, Cline HH, Jolesz FA. MR monitoring of focused ultrasonic surgery of renal cortex: experimental and simulation studies. J. Magn. Reson. Imag. 1995; 5(3):259-66.
8. Christakis Damianou, M. Pavlou, O. Velev, K. Kyriakou, M. Trimikliniotis 'High intensity focused ultrasound ablation of kidney guided by MRI', Journal of Ultrasound in Medicine and Biology Vol. 30 (3), pp. 397-404, 2004
9. Hynynen K, Pomeroy O, Smith DN,. Huber PE, McDannold NJ, Kettenbach J, Baum J, Singer S, Jolesz FA. MR imaging-guided focused ultrasound surgery of fibroadenomas in the breast: a feasibility study. Radiology 2001; 219(1):176-85.
10. Jolesz FA, Jakab PD. Acoustic pressure wave generation within a magnetic resonance imaging system: potential medical applications. J. Magn. Reson. Imag. 1991; 1(5):609-13.
11. Hynynen K, Darkazanli A, Unger E, Schenck JF. MRI-guided noninvasive ultrasound surgery. Med. Phys. 1993; 20(1):107-15.
12. Ettinger R, Cline H, Watkins R, Rohling K, inventors General Electric Company, assignee. Magnetic resonance guided ultrasound therapy system with inclined track to move transducers in a small vertical space. United States patent US5275165. 1994.
13. Cline H, Rohling K, Abeling W, inventors General Electric Company, assignee. Mechanical positioner for magnetic resonance guided ultrasound therapy. United States patent US5443068. 1995.
14. Yehezkeli O, Freundlich D, Magen N, Marantz C, Medan Y, Vitek S, Weinreb A, inventors, INSIGHTEC-TXSONICSLTD, assignee. Mechanical positioner for MRI guided ultrasound therapy system WO0209812. 2002.
15. Stewart EA, Gedroyc W, Tempany CM, Quade B, Inbar Y, Ehrenstein T, Shushan A, Hindley J, Goldin R, David M, Sklair M, Rabinovici J. Focused Ultrasound Treatment of Uterine Fibroids: Safety and Feasibility of a Noninvasive Thermoablative Technique, Am J Obstet Gynecol, 2003, pp. 48-54.
16. Dorenberg EJ, Courivaud F, Ring E, Hald K, Jakobsen JÅ, Fosse E, Hol PK. 'Volumetric ablation of uterine fibroids using Sonalleve high-intensity focused ultrasound in a 3 Tesla scanner--first clinical assessment', Minim Invasive Ther Allied Technol. 2013 Apr;22(2):73-9.
17. Chopra R., Curiel L., Staruch R., Morrison L., Hynynen K., 'An MRI-compatible system for focused ultrasound experiments in small animal models' , Med. Phys. 36 (5),1867-1874May 2009.
18. C. Damianou, N. Milonas, K. Ioannides, (2008). Positioning Device for MRI-guided high intensity focus ultrasound system, CARS (2008) 2:335-345.
19. Bihrle R, Foster RS, Sanghvi NT, et al. High-intensity focused ultrasound in the treatment of prostatic tissue. Urology 1994;43(2 Suppl):21-6.
20. Gelet A, Chapelon JY, Margonari J, et al. Highintensity focused ultrasound experimentation on human benign prostatic hypertrophy. Eur Urol 1993;23(Suppl 1):44-7.
21. Madersbacher S, Pedevilla M, Vingers L, et al. Effect of high-intensity focused ultrasound on human prostate cancer in vivo. Cancer Res 1995; 55(15):3346-51.
22. Chaussy C, Thuroff S. The status of high-intensity focused ultrasound in the treatment of localized prostate cancer and the impact of a combined resection. Curr Urol Rep 2003;4(3):248-52.
23. Madersbacher S, Marberger M. High-energy shockwaves and extracorporeal high-intensity focused ultrasound. J Endourol 2003;17(8): 667-72.
24. Saleh KY, Smith NB. A 63 element 1.75 dimensional ultrasound phased array for the treatment of benign prostatic hyperplasia. Biomed Eng Online 2005;4(1):39.
25. Hacker A, Ko"hrmann KU, Back W, et al. Extracorporeal application of high-intensity focused ultrasound for prostatic tissue ablation. BJU Int 2005; 96(1):71-6.

## Claims

1. A robotic system for navigating a focused ultrasound (FUS) transducer (41) under magnetic resonance imaging guidance for treating fibroids, breast, abdominal and brain tumor targets comprising:
the FUS transducer (41);
a FUS transducer holder (42, 48);
a first stage (1-9) dedicated to control the position of the FUS transducer in the X axis;
a second stage (10-21) dedicated to control the position of the FUS transducer in the Y axis;
a third stage (22-31) dedicated to control the position of the FUS transducer in the Z axis;
a fourth stage (32-40) dedicated to control the position of the FUS transducer in an angular axis;
and a water container (49) for coupling the FUS transducer (41) to the fibroid, breast, abdominal and brain tumor targets.

2. The robotic system of claim 1, wherein the first stage for moving the FUS transducer in the X direction comprises:
a first piezoelectric motor (1);
a first holder (8) supporting the first motor; a first jack screw (3) coupled to the first motor;
and a first bracket (5) coupled with a coupling mechanism to the first jack screw, the first bracket being adapted to carry an X-plate (10);
and a rectangular base (2) that carries the above components.

3. The robotic system of claim 2, wherein the second stage for moving the FUS transducer in the Y direction comprises:
the X-plate (10) carried by the first bracket (5);
a Y-plate (18); a second piezoelectric motor (11) a second jack screw (14) coupled to the second motor (11);
a second holder supporting the second motor;
a rectangular second bracket (17) coupled with a coupling mechanism to the second jack screw, the second bracket being adapted to carry the Y-plate (18);
and a first coupling (21) to attach the third stage.

4. The robotic system of claim 3, wherein the third stage for moving the transducer in the Z direction comprises:
a frame (23) attachable to the first coupling (21);
a third piezoelectric motor (22);
a third jack screw (24) coupled to the third motor;
a third holder supporting the third motor;
and a second coupling (16) to attach the fourth stage.

5. The robotic system of claim 4, wherein the fourth stage comprises:
a fourth piezoelectric motor (33, 43);
an angular arm (37, 38, 46, 47) coupled to the fourth motor;
wherein the fourth stage carries the FUS transducer (41) and the FUS transducer holder (42, 48);
and wherein the fourth stage is attachable to the third stage by the second coupling (26).

6. The robotic system of claim 5, wherein the fourth stage that is adapted to move the FUS transducer (41) at an angle (-90 ° to +90 °) at a plane perpendicular to the base (2)
or is adapted to move the FUS transducer at an angle (-90 ° to +90 °) at a plane parallel to the base (2).

7. The robotic system of claim 1 wherein in use the water container (49) is filled with degassed water so that ultrasound energy is acoustically coupled to the target and wherein, in use, the FUS transducer (41) is immersed in the water container.

8. The robotic system of claim 7, further comprises an ultrasound transparent membrane attached to the water container and adapted for contact with the target.

9. The robotic system of claim 1 being placeable on the table of an MRI scanner adapted to access to a patient that is positioned either in supine or prone positions.

10. The robotic system of claim 1, adapted to access targets from the bottom to top or top to bottom for fibroids, brain, abdominal and breast tumor targets or laterally for brain tumor targets.

11. The robotic device of claim 1, guidable by magnetic resonance imaging (MRI) comprising:
a magnetic resonance (MR) imaging means for imaging brain, breast, fibroid, and abdominal sites and for creating temperature sensitive images of ablated sites during surgery;
a flex coil which is placeable around the patients target and allows an energy source to be coupled to the patient's head.

12. The robotic system of claim further comprising MRI compatible linear encoders (6, 16, 29).

13. The robotic system of claim 1 further comprises an MRI compatible angular encoder (48).

## Patentansprüche

1. Robotersystem zum Navigieren eines fokussierten Ultraschallwandlers (FUS) unter Magnetresonanz-Bildgebungsführung zur Behandlung von Myomen, Brust-, Bauch- und Hirntumoren, umfassend:
der FUS-Wandler (41);
einen FUS-Wandlerhalter (42, 48);
eine erste Stufe (1-9) zur Steuerung der Position des FUS-Wandlers in der X-Achse;
eine zweite Stufe (10-21) zur Steuerung der Position des FUS-Wandlers in der Y-Achse;
eine dritte Stufe (22-31) zur Steuerung der Position des FUS-Wandlers in der Z-Achse;
eine vierte Stufe (32-40), die dazu bestimmt ist, die Position des FUS-Wandlers in einer Winkelachse zu steuern;
und einen Wasserbehälter (49) zum Koppeln des FUS-Wandlers (41) an die Myom-, Brust-, Bauch- und Hirntumorziele.

2. Robotersystem nach Anspruch 1, wobei die erste Stufe zum Bewegen des FUS-Wandlers in der X-Richtung umfasst:
;ein erster piezoelektrischer Motor (1);
einen ersten Halter (8), der den ersten Motor trägt;
eine erste Stellschraube (3), die mit dem ersten Motor gekoppelt ist;
und eine erste Klammer (5), die mit einem Kopplungsmechanismus an die erste Zylinderschraube gekoppelt ist, wobei die erste Klammer angepasst ist, um eine X-Platte (10) zu tragen;
und eine rechteckige Basis (2) das trägt die oben genannten Komponenten.

3. Robotersystem nach Anspruch 2, wobei die zweite Stufe zum Bewegen des FUS-Wandlers in der Y-Richtung umfasst:
die von der ersten Halterung (5) getragene X-Platte (10);
eine Y-Platte (18); einen zweiten piezoelektrischen Motor (11), eine zweite Stellschraube (14), die mit dem zweiten Motor (11) gekoppelt ist;
einen zweiten Halter, der den zweiten Motor trägt;
eine rechteckige zweite Klammer (17), die mit einem Kopplungsmechanismus mit der zweiten Zylinderschraube gekoppelt ist, wobei die zweite Klammer die Y-Platte (18) tragen kann;
und eine erste Kupplung (21) zum Anbringen der dritten Stufe.

4. Robotersystem nach Anspruch 3, wobei die dritte Stufe zum Bewegen des Wandlers in der Z-Richtung umfasst:
a einen Rahmen (23), der an der ersten Kupplung (21) anbringbar ist;
einen dritten piezoelektrischen Motor (22);
eine dritte Stellschraube (24), die mit dem dritten Motor gekoppelt ist;
einen dritten Halter, der den dritten Motor trägt;
und eine zweite Kupplung (16) zum Anbringen der vierten Stufe.

5. Robotersystem nach Anspruch 4, wobei die vierte Stufe umfasst:
einen vierten piezoelektrischen Motor (33, 43);
einen Winkelarm (37, 38, 46, 47), der mit dem vierten Motor gekoppelt ist;
wobei die vierte Stufe den FUS-Wandler (41) und den FUS-Wandlerhalter (42, 48);
trägtund wobei die vierte Stufe durch die zweite Kupplung (26) an der dritten Stufe anbringbar ist.

6. Robotersystem nach Anspruch 5, wobei die vierte Stufe den FUS-Wandler (41) in einem Winkel (-90 ° bis +90 °) in einer Ebene senkrecht zur Basis (2)
bewegen kann.oder dazu ausgelegt ist, den FUS-Wandler in einem Winkel (-90 ° bis +90 °) in einer Ebene parallel zur Basis (2) zu bewegen.

7. Robotersystem nach Anspruch 1, bei dem das Wasser verwendet wird Der Behälter (49) ist mit entgastem Wasser gefüllt, so dass Ultraschallenergie akustisch an das Ziel gekoppelt wird und der FUS-Wandler (41) im Gebrauch in den Wasserbehälter eingetaucht ist.

8. Robotersystem nach Anspruch 7, ferner umfassend eine ultraschalltransparente Membran, die an dem Wasserbehälter angebracht und für den Kontakt mit dem Ziel angepasst ist.

9. Robotersystem nach Anspruch 1, das auf dem Tisch eines MRT-Scanners plazierbar ist, der für den Zugang zu einem Patienten geeignet ist, der entweder in Rückenlage oder in Bauchlage positioniert ist.

10. Robotersystem nach Anspruch 1, angepasst, um auf Ziele von unten nach oben oder von oben nach unten für Myome, Gehirn-, Bauch- und Brusttumorziele oder seitlich für Hirntumorziele zuzugreifen.

11. Robotervorrichtung nach Anspruch 1, steuerbar durch Magnetresonanztomographie (MRT), umfassend:ein Magnetresonanz (MR) -Bildgebungsmittel zum Abbilden von Gehirn-, Brust-, Myom- und Bauchstellen und zum Erzeugen temperaturempfindlicher Bilder von abgetragenen Stellen während der Operation;
eine flexible Spule, die um das Ziel des Patienten platziert werden kann und die es ermöglicht, eine Energiequelle an den Kopf des Patienten zu koppeln.

12. Robotersystem nach Anspruch 1, ferner mit MRI-kompatiblen linearen Codierern (6, 16, 29).

13. Robotersystem nach Anspruch 1, ferner umfassend einen MRI-kompatiblen Winkelcodierer (48).

## Revendications

1. Système robotique de navigation dans un transducteur d'ultrasons focalisés (FUS) sous guidage d'imagerie par résonance magnétique pour le traitement de fibromes, de cibles de tumeurs du sein, de l'abdomen et du cerveau, comprenant:
le transducteur FUS (41);
un support de transducteur FUS (42, 48);
une première étape (1-9) dédiée au contrôle de la position du transducteur FUS sur l'axe X;
un deuxième étage (10-21) dédié à la commande de la position du transducteur FUS dans l'axe des Y;
une troisième étape (22-31) dédiée au contrôle de la position du transducteur FUS dans l'axe Z;
un quatrième étage (32-40) dédié au contrôle de la position du transducteur FUS dans un axe angulaire;
et un réservoir d'eau (49) pour coupler le transducteur de FUS (41) aux cibles des tumeurs du fibrome, du sein, de l'abdomen et du cerveau.

2. Système robotique selon la revendication 1, dans lequel le premier étage pour déplacer le transducteur FUS dans la direction X comprend:
un premier moteur piézoélectrique (1);
un premier support (8) supportant le premier moteur;
une première vis creuse (3) couplée au premier moteur;
et un premier support (5) couplé à un mécanisme de couplage à la première vis à cric, le premier support étant adapté pour porter une plaque en X (10);
et une base rectangulaire (2) qui porte les composants ci-dessus.

3. Système robotique selon la revendication 2, dans lequel le deuxième étage pour déplacer le transducteur FUS dans la direction Y comprend:
la plaque en X (10) portée par le premier support (5);
une plaque en Y (18); un deuxième moteur piézoélectrique (11), une deuxième vis à cric (14) couplée au deuxième moteur (11);
un second support supportant le second moteur;
un second support rectangulaire (17) couplé à un mécanisme de couplage à la seconde vis à cric, le second support étant adapté pour porter la plaque en Y (18);
et un premier couplage (21) pour attacher le troisième étage.

4. Système robotique selon la revendication 3, dans lequel le troisième étage pour déplacer le transducteur dans la direction Z comprend:
un cadre (23) pouvant être fixé au premier couplage (21);
un troisième moteur piézoélectrique (22);
une troisième vis à cric (24) couplée au troisième moteur;
un troisième support supportant le troisième moteur;
et un second couplage (16) pour attacher le quatrième étage.

5. Système robotique selon la revendication 4, dans lequel la quatrième étape comprend:
un quatrième moteur piézoélectrique (33, 43);
un bras angulaire (37, 38, 46, 47) couplé au quatrième moteur;
dans lequel le quatrième étage porte le transducteur FUS (41) et le support de transducteur FUS (42, 48)et dans lequel le quatrième étage est attachable au troisième étage par le deuxième couplage (26).

6. Système robotique selon la revendication 5, dans lequel le quatrième étage est adapté pour déplacer le transducteur de FUS (41) selon un angle (-90 o à +90 o) dans un plan perpendiculaire à la base (2)
ou est adapté pour déplacer le transducteur FUS selon un angle (-90 o à +90 o) dans un plan parallèle à la base (2).

7. Système robotique selon la revendication 1, dans lequel, pendant l'utilisation, le réservoir d'eau (49) est rempli d'eau dégazée, de sorte que l'énergie ultrasonore est couplée de manière acoustique à la cible et dans lequel, lors de l'utilisation, le transducteur FUS (41) est immergé dans le réservoir d'eau.

8. Système robotique selon la revendication 7, comprenant en outre une membrane transparente aux ultrasons fixée au réservoir d'eau et adaptée pour le contact avec la cible.

9. Système robotique selon la revendication 1, pouvant être placé sur la table d'un scanner IRM adapté pour accéder à un patient qui est positionné en position couchée ou couchée.

10. Système robotique selon la revendication 1, adapté pour accéder à des cibles de bas en haut ou de haut en bas pour des cibles de fibromes, de tumeurs cérébrales, abdominales et mammaires ou latéralement pour des cibles de tumeurs cérébrales.

11. Dispositif robotique selon la revendication 1, pouvant être guidé par imagerie par résonance magnétique (IRM) comprenant:un moyen d'imagerie par résonance magnétique (IRM) pour imager les sites du cerveau, du sein, des fibromes et de l'abdomen et pour créer des images sensibles à la température des sites sous ablation pendant une intervention chirurgicale;
une bobine flexible qui peut être placée autour de la cible du patient et permet à une source d'énergie d'être couplée à la tête du patient.

12. Système robotique selon la revendication 1, comprenant en outre des codeurs linéaires compatibles avec l'IRM (6, 16, 29).

13. Système robotique selon la revendication 1, comprenant en outre un codeur angulaire compatible avec l'IRM (48).
